# FASCICULE DE BREVET EUROPEEN

(11) **EP 1 743 671 B1**
(45) Date de publication et mention de la délivrance du brevet: **11.12.2013**
(21) Numéro de dépôt: 06300795.9
(22) Date de dépôt: 12.07.2006
(51) Int. Cl.: A61M 15/00

(54) **Appareil pour l'administration d'un aérosol médicamenteux**
Vorrichtung zum Verabreichen eines medizinischen Aerosols
Device for administering a medicinal aerosol

(30) Priorité: 13.07.2005 FR 0552184
(43) Date de publication de la demande: 17.01.2007
(73) Titulaire: LA DIFFUSION TECHNIQUE FRANCAISE, 42000 Saint-Etienne (FR)
(72) Inventeur: Vecellio-None, Laurent, 31170 Chambray Les Tours (FR); Massardier, Michel, 42000 Saint Etienne (FR); Chantrel, Gilles, 42100 Saint-Etienne (FR)
(74) Mandataire: Dupuis, François

(56) Documents cités:
- EP-A1- 0 682 955
- EP-A2- 0 251 443
- GB-A- 2 233 919
- GB-A- 2 334 217
- US-A- 4 173 977
- US-A1- 2002 104 531
- US-B1- 6 363 932

## Description

L'invention se rattache au secteur technique des systèmes de génération d'aérosols médicaux.

Les systèmes de génération d'aérosols médicaux ont pour fonction de transformer, un liquide ou une poudre, médicamenteux sous forme d'aérosol pour être administré dans les voies aériennes respiratoires.

Divers systèmes de génération d'aérosols médicaux existent sur le marché avec des sous formes d'appareils à commandes pneumatiques, ultra soniques, à membrane vibrante, notamment, ainsi que des flacons pressurisés avec valve doseuse.

Le Déposant est l'un des leaders dans la fabrication et la commercialisation de ce type d'appareils. Malgré tous les efforts de recherche en rapport avec ces appareils, générateurs, nébuliseurs, flacons pressurisés, les différentes études publiées en rapport sont toutes d'accord pour constater qu'une large part de l'aérosol projeté est perdue, gaspillée et ne profite pas au traitement thérapeutique souhaité. La part d'utilisation de l'aérosol dans sa fonction est ainsi estimée par les auteurs et fabricants à 25 % environ, ce qui est couramment explicité par la fraction inhalable ou disponible. La déperdition est due à plusieurs causes dont la perte de médicament dans l'atmosphère durant la phase expiratoire du patient. L'explication qui suit est la conséquence directe d'un problème précédemment posé dans la demande de brevet FR 04 13784 appartenant au présent Demandeur dans l'exemple de la cinquième configuration utilisant une chambre d'inhalation sans système de valves.

Les chambres d'inhalation actuelles qui fonctionnent sans valves ne limitent pas la perte de médicament dans l'atmosphère durant la phase expiratoire du patient. En effet, lorsque le patient expire dans la chambre d'inhalation, l'air expiré par le patient traverse la chambre d'inhalation. L'aérosol est alors expulsé hors de la chambre d'inhalation et est perdu. Une autre conséquence de ce système est la contamination de la chambre par l'air expiré. En effet, lorsque le patient expire ou tousse dans la chambre, il contamine l'intérieur de la chambre d'inhalation.

Pour palier au problème de perte d'aérosol durant la phase expiratoire, différentes chambres d'inhalation utilisent un système de valves. Ce système de valves peut être utilisé de deux façons différentes, chacune présentant des avantages et inconvénients. La première façon d'utiliser le système de valves, est de placer une valve sur la sortie de l'aérosol, en face avant de la chambre d'inhalation, près du patient. Dans ces conditions, lorsque le patient expire, la valve se ferme et l'air exhalé ne traverse pas la chambre d'inhalation mais est expulsé hors du système. Lorsque le patient inspire, la valve s'ouvre pour laisser passer l'aérosol vers la bouche du patient. Ce système possède l'avantage de ne pas contaminer la chambre d'inhalation par l'air exhalé du patient mais possède l'inconvénient de pertes importantes de médicaments au niveau de la valve. En effet, l'emplacement de la valve sur le trajet de l'aérosol piège des particules de médicament, diminuant ainsi le rendement du système.

Dans la seconde mise en oeuvre, le deuxième système de valves permet de diminuer les pertes de médicaments au niveau de la valve. Il consiste à utiliser un système de deux valves. Une première valve inspiratoire est placée sur la partie arrière de la chambre d'inhalation et une deuxième valve expiratoire est placée sur la partie avant de la chambre d'inhalation, près du patient. Les valves ne sont pas sur le trajet de l'aérosol. Dans ces conditions, lorsque le patient expire, la valve inspiratoire se ferme et la valve expiratoire s'ouvre, l'air exhalé ne traverse pas la chambre d'inhalation où est stocké l'aérosol mais est expulsé hors du système. Lorsque le patient inspire, la valve inspiratoire s'ouvre et la valve expiratoire se ferme. L'air entrant par la valve inspiratoire en arrière de la chambre traverse la chambre d'inhalation pour transporter l'aérosol vers le patient. Contrairement au système de valves précédent, ce système possède l'avantage de ne pas être la cause d'une nouvelle perte de médicament sur les valves. Par contre ce système à l'inconvénient d'une contamination potentielle de la chambre d'inhalation. Par exemple, lorsque le patient tousse, la résultante de la toux contaminera directement l'intérieur de la chambre d'inhalation. De plus, ce système de valve nécessite l'utilisation d'un volume clos permettant l'ouverture et la fermeture des valves par la respiration du patient. Ce qui dans le cas des flacons doseur pressurisé, nécessite une modification de leur conditionnement pour l'adapter à ce système de valves.
En conclusion, les chambres d'inhalation utilisant un système de valves permettent de limiter les pertes d'aérosols durant l'expiration du patient, par contre, elles ne permettent pas d'assurer simultanément la non-contamination de la chambre par le patient et la non-perte de médicament au niveau des valves.

En effet, dans tous les systèmes actuels, la zone qui transporte l'aérosol depuis la chambre d'inhalation vers le patient est conçue de façon longitudinale (sans coude) pour limiter la perte de l'aérosol par dépôt sur les parois (impaction de l'aérosol). L'utilisation de cette zone de transport longitudinale nécessite un système de valves pour limiter la perte de médicament dans l'atmosphère durant la phase expiratoire du patient. En effet, si la chambre d'inhalation ne possède pas de valves, l'air expiré par le patient traverse la chambre d'inhalation.

Le document US 4,173,977 représente l'état de la technique le plus pertinent, et décrit un appareil pour l'administration d'un aérosol médicamenteux selon le préambule de la revendication 1. Cet appareil comprend un générateur d'aérosol médicamenteux, une chambre d'inhalation sans valve constituant une zone de stockage de l'aérosol produit notamment pendant la phase d'expiration du patient par le générateur d'aérosol, et un dispositif de transfert d'aérosol sans valve constituant une zone de transport de l'aérosol depuis la chambre d'inhalation vers le patient.

La démarche du Demandeur a donc été de reconsidérer le problème de cette perte de médicament dans l'atmosphère durant la phase expiratoire du patient et de rechercher une nouvelle solution sans l'utilisation de valves afin de limiter les coûts de fabrication et d'améliorer les conditions de traitement thérapeutique des patients.

Face à cette situation, le Demandeur s'est alors orienté sur une conception différente de ce type d'appareils.

La présente invention propose ainsi un appareil pour l'administration d'un aérosol médicamenteux comprenant:
- un générateur d'aérosol médicamenteux,
- une chambre d'inhalation sans valve constituant une zone de stockage de l'aérosol produit notamment pendant la phase d'expiration du patient par le générateur d'aérosol, et
- un dispositif de transfert d'aérosol sans valve constituant une zone de transport de l'aérosol depuis la chambre d'inhalation vers le patient,
- la chambre d'inhalation comprenant au minimum deux ouvertures dont la première est obligatoirement destinée à être connectée au dispositif de transfert d'aérosol et la deuxième permet au minimum le passage de l'air extérieur à travers la chambre d'inhalation lors de la phase inspiratoire du patient pour assurer le transfert de l'aérosol au patient,
- le dispositif de transfert d'aérosol, situé entre la chambre d'inhalation et le patient lors de l'utilisation de l'appareil, comprenant au minimum trois ouvertures, une première ouverture connectée à la première ouverture de la chambre d'inhalation, une seconde ouverture destinée à être connectée au patient, et une troisième ouverture étant libre et disposée à l'opposé de la seconde ouverture dans l'axe de celle-ci, ladite première ouverture se trouvant positionnée entre, et en communication avec, la seconde et la troisième ouverture, l'axe de la première ouverture formant un angle de 45° à 135° avec l'axe reliant les deux autres ouvertures de manière à définir un coude dont la fonction est de guider et d'orienter les flux d'air en cas d'expiration et d'inspiration du patient de manière différenciée, la disposition des trois ouvertures ayant pour fonction d'autoriser le transfert de l'air expiré par le patient entre la seconde ouverture et la troisième ouverture située dans l'axe rectiligne du dispositif de transfert d'aérosol, l'air expiré depuis la seconde ouverture étant expulsé directement vers la troisième ouverture, et en cas d'inspiration par le patient, d'autoriser un flux d'air entrant par la troisième ouverture et par la première ouverture de la chambre d'inhalation.

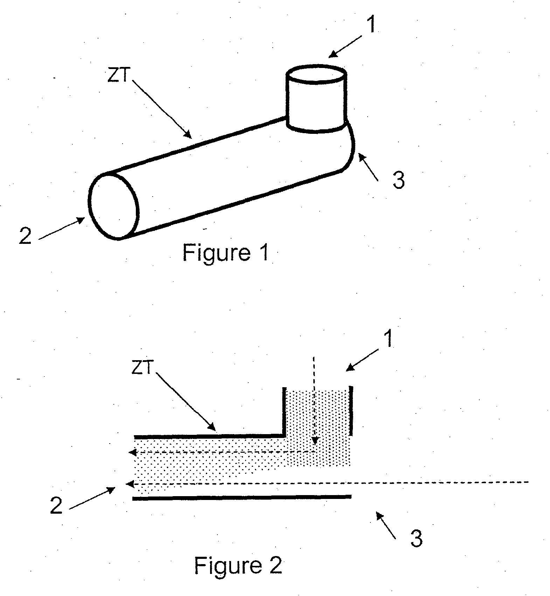

Dans une mise en oeuvre particulière, le dispositif de transfert d'aérosol constituant la zone de transport (ZT) possède trois ouvertures (figures 1 et 2), la première ouverture (1) étant destinée à recevoir la chambre d'inhalation constituant la zone de stockage (ZS) en formant une angulation par exemple de l'ordre de 90° avec l'axe reliant les deux autres ouvertures (2 et 3), la distance entre l'ouverture (2) destinée a être connecté au patient et l'ouverture (1) destinée à recevoir la chambre d'inhalation (ZS) (1) étant plus importante que la distance entre la troisième ouverture (3) située en face de l'ouverture (2) et l'ouverture (1), l'ouverture (1) étant placée perpendiculairement et juste à coté de l'ouverture (3), ces deux ouvertures (1 et 3) possédant la même surface. Dans cette mise en oeuvre, l'air expiré par le patient ne traverse pas la chambre d'inhalation. L'air expiré par le patient passe préférentiellement par l'ouverture (3) située dans l'axe longitudinal de la zone de transport (ZT), car le dard du jet d'air expiré par le patient est lui aussi longitudinal et l'ouverture (3) est en communication avec l'ouverture (2) qui offre un accès direct et plus ouvert que l'accès à l'ouverture (1) recevant la chambre d'inhalation.

Lorsque le patient inspirera dans la zone de transport (ZT) par l'ouverture (2), la dépression créée par le patient générera un flux d'air entrant non seulement par l'ouverture (3) mais également par l'ouverture (1) d'accès à la chambre d'inhalation. En effet, dans ces conditions, les lignes de dépression de l'aspiration décrivent un cercle autour de la source d'aspiration (il n'y a pas de dard de jet d'air). En conséquence, le dispositif de transfert d'aérosol constituant la zone de transport (ZT) permet que l'air expiré par le patient ne traverse pas la chambre d'inhalation mais l'air inspiré par le patient traverse la chambre d'inhalation.

Le dispositif de transfert d'aérosol constituant la zone de transport (ZT) est également remarquable en ce qu'il ne crée pas d'impaction de particules dans le coude de la zone de transport (ZT). En effet, lors de l'inspiration du patient, l'air extérieur entrant par l'ouverture (3) longe le bord de la zone de transport (ZT) opposé à l'ouverture (1) empêchant ainsi aux particules qui arrivent transversalement en provenance de l'ouverture (1) de s'impacter sur la zone de transport (ZT). L'air arrivant par l'ouverture (3) agit comme un écran d'air pour protéger la zone de transport (ZT) de l'impaction.
Ainsi l'angle décrit par le dispositif de transfert d'aérosol constituant la zone de transport (ZT) permet un fonctionnement du système équivalent à un système de valves sans dépôt par impaction dans la zone de transport (ZT).
Le dispositif de transfert d'aérosol constituant la zone de transport (ZT) cumule les avantages du système de valves sans leurs inconvénients. En effet, le dispositif permet de limiter les pertes d'aérosols durant l'expiration du patient, tout en assurant la non-contamination de la chambre par le patient et la non-perte de médicament au niveau de la zone de transport (ZT).

Ces caractéristiques et d'autres encore ressortiront bien de la suite de la description.
- La figure 1 illustre une zone de transport en perspective pour l'utilisation d'une interface patient de type embout buccal.
- La figure 2 illustre la zone de transport de la figure 1 en vue de section.
- Les figures 3, 4 et 5 sont des vues en perspective de la configuration de la zone de transport assimilée à l'utilisation d'un masque facial.
- La figure 6 est une vue en perspective de la zone de transport dans le cas de son utilisation avec une zone de stockage utilisée horizontalement et connectée en son extrémité latérale.
- Les figures 7 et 8 sont des vues sagittales de la zone de transport dans le cas de son utilisation avec une zone de stockage utilisée horizontalement et connectée en son extrémité latérale.
- La figure 9 est une vue en perspective de la zone de transport dans le cas de son utilisation avec une zone de stockage utilisée verticalement et connectée en son extrémité basse.
- Les figures 10 et 11 sont des vues de section de la zone de transport dans le cas de son utilisation avec une zone de stockage utilisée verticalement et connectée en son extrémité basse.
- La figure 12 est une vue en perspective de la zone de transport dans le cas de son utilisation avec une zone de stockage utilisée verticalement et connectée sur le haut de sa face latérale.
- Les figures 13 et 14 sont des vues de section latérale de la zone de transport dans le cas de son utilisation avec une zone de stockage utilisée verticalement et connectée sur le haut de sa face latérale.
- Les figures 15 et 16 sont des vues sagittales de la zone de transport dans le cas de son utilisation avec une zone de stockage utilisée verticalement et connectée sur le haut de sa face latérale.
- La figure 17 est une vue en perspective de la zone de transport dans le cas de son utilisation avec une zone de stockage utilisée horizontalement et connectée sur sa face supérieure.
- Les figures 18 et 19 sont des vues de section de la zone de transport dans le cas de son utilisation avec une zone de stockage utilisée horizontalement et connectée sur sa face supérieure.
- La figure 20 est une vue en perspective de la zone de transport dans le cas de son utilisation avec une zone de stockage utilisée verticalement et connectée sur sa face supérieure.
- Les figures 21 et 22 sont des vues de section de la zone de transport dans le cas de son utilisation avec une zone de stockage utilisée verticalement et connectée sur sa face supérieure.
- La figure 23 est une vue en perspective de la zone de transport dans le cas de son utilisation avec une zone de stockage horizontale possédant uniquement deux ouvertures et connectée en son extrémité latérale.
- Les figures 24 et 25 sont des vues sagittales de la zone de transport dans le cas de son utilisation avec une zone de stockage horizontale possédant uniquement deux ouvertures et connectée en son extrémité latérale.
- La figure 26 est une vue en perspective de la zone de transport dans le cas de son utilisation avec une zone de stockage, utilisée verticalement, et contenue dans la zone de stockage.
- Les figures 27 et 28 sont des vues de section de la zone de transport dans le cas de son utilisation avec une zone de stockage utilisée verticalement, et contenue dans la zone de stockage.

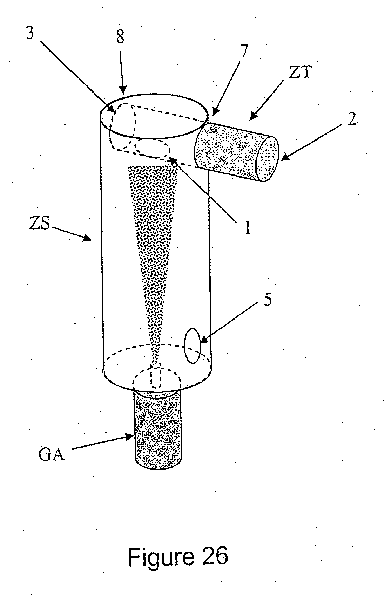

Les figures 26, 27 et 28 illustrent un mode de réalisation qui ne fait pas partie de la présente invention.

Les systèmes de génération d'aérosol médicaux sont composés d'un générateur d'aérosol à proprement dit et d'une interface entre le générateur d'aérosol et le patient. Le générateur d'aérosol est la source de génération de l'aérosol. L'interface patient - générateur d'aérosol permet le transport de l'aérosol, depuis le générateur vers le patient (exemples : masque, embout buccal, embout narinaire, circuit de ventilateur mécanique, sonde d'intubation, sonde trachéale...). L'appareil pour l'administration d'un aérosol médicamenteux selon l'invention comprend une chambre d'inhalation sans valve constituant une zone de stockage (ZS) entre la zone de génération de l'aérosol et l'interface. Cette zone de stockage (ZS) est destinée à stocker l'aérosol durant l'expiration du patient. L'appareil selon l'invention comprend aussi un dispositif de transfert d'aérosol sans valve constituant une zone de transport (ZT) entre la zone de stockage de l'aérosol et l'interface patient.

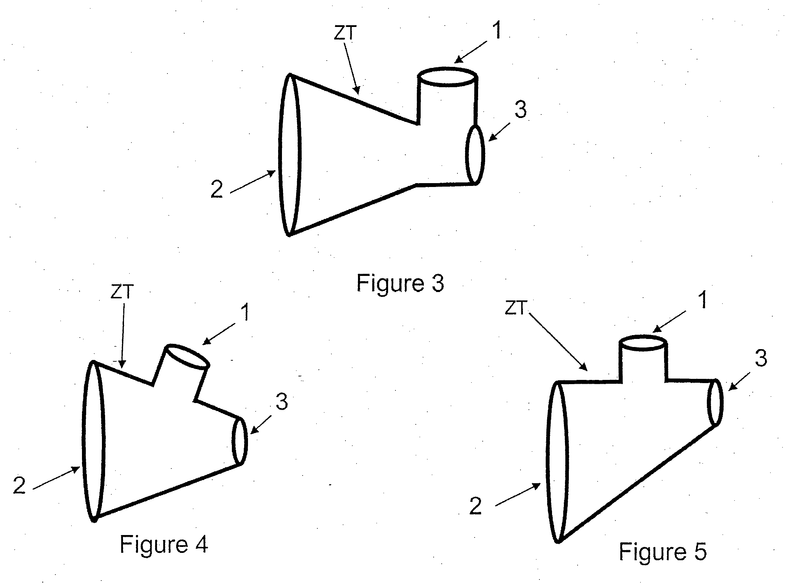

Cette zone de transport (ZT) est destinée à transporter l'aérosol généré par le générateur d'aérosol, puis par l'intermédiaire de l'interface patient-générateur à le transporter hors du système de génération d'aérosol. Cette zone de transport (ZT) peut faire partie intégrante de l'interface (Figures 3, 4 et 5) ou être indépendant (Figure 1).

Le dispositif de transfert d'aérosol constituant la zone de transport (ZT) contient trois ouvertures.

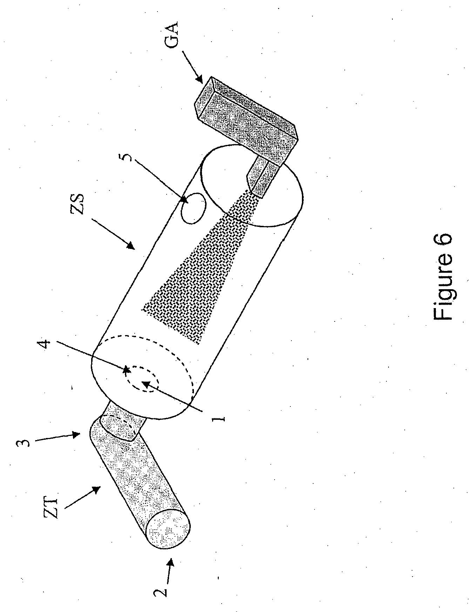

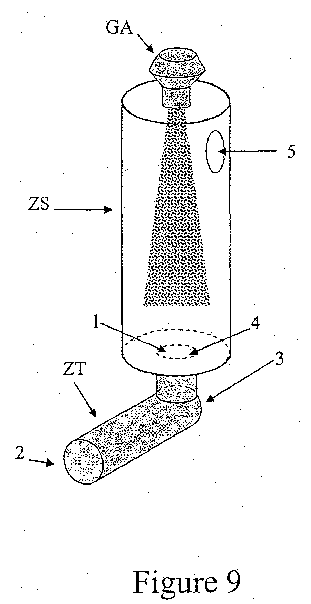

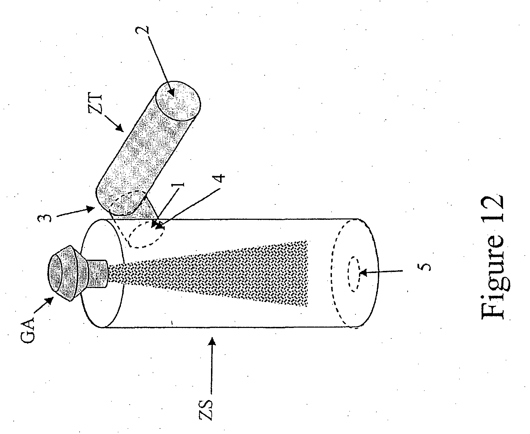

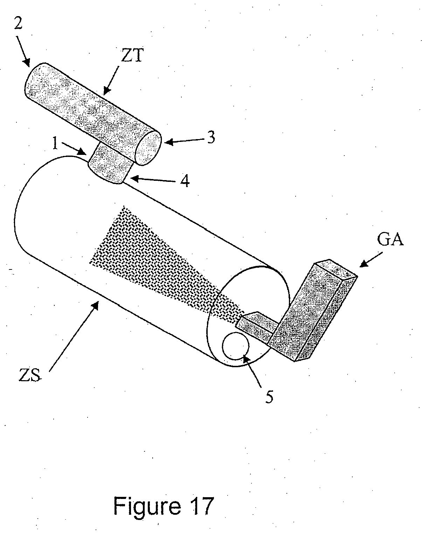

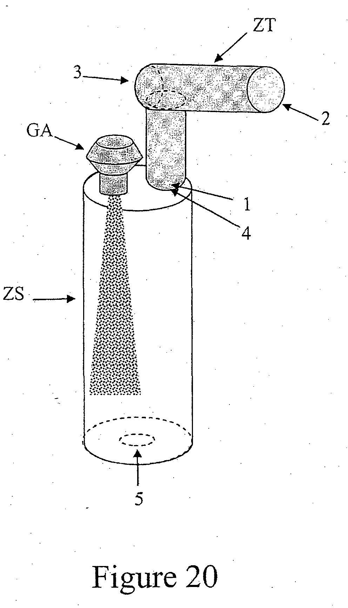

La première ouverture (1) est destinée à recevoir la zone de stockage (ZS). L'axe de cette ouverture forme un angle de 45° à 135° avec l'axe reliant les deux autres ouvertures (2-3), de manière à définir un coude dont la fonction est de guider et d'orienter les flux d'air en cas d'expiration et d'inspiration du patient de manière différenciée. La deuxième ouverture (2) destinée à être connectée au patient permet au minimum le passage de l'air inspiré depuis la zone de transport (ZT) vers le patient et le passage de l'air expiré depuis le patient vers la zone de transport (ZT). La troisième ouverture (3) située en face de la deuxième ouverture (2) permet au minimum le passage de l'air inspiré depuis l'extérieur vers la zone de transport(ZT) et le passage de l'air expiré depuis la zone de transport (ZT) vers l'extérieur. Cette ouverture (3) est libre.
Cette zone de transport (ZT) peut avoir une section quelconque. Dans une configuration avantageuse, elle peut avoir une section circulaire et une distance entre l'ouverture (3) et l'ouverture (1) moins importante que la distance entre l'ouverture (2) et l'ouverture (1) (figures 1 et 2). Dans une autre configuration, notamment lorsqu'elle fait partie intégrante de l'interface patient et qu'elle est destinée à être utilisée en masque facial, elle peut avoir une section conique (figures 3, 4 et 5).
Cette zone de transport (ZT) peut être placée sur toutes les faces à un endroit quelconque de la zone de stockage (ZS). Dans une des configurations, la zone de transport (ZT) peut être placée sur le bas de la zone de stockage (ZS) (figure 9), sur le haut de la zone de stockage (ZS) (figures 17 et 20), sur la face latérale de la zone de stockage (ZS) (figures 6 et 12).

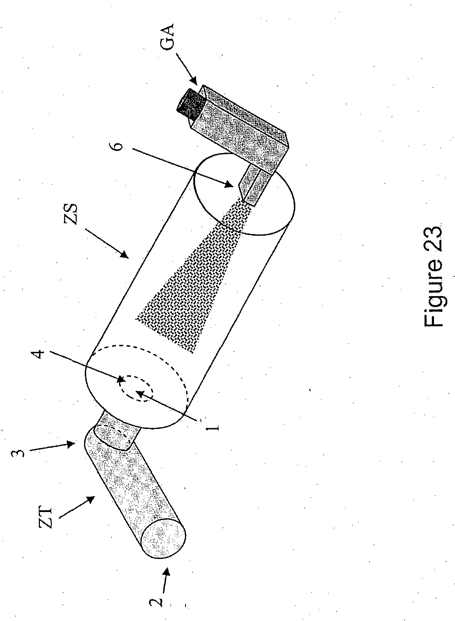

La zone de stockage (ZS) entre la zone de transport (ZT) et le générateur d'aérosol doit contenir au minimum deux ouvertures. Une des ouvertures est obligatoirement destinée à être connectée avec la zone de transport (ZT) et l'autre ouverture permet au minimum le passage de l'air extérieur à travers la zone de stockage (ZS). Cette autre ouverture peut aussi recevoir un générateur d'aérosol contenant lui-même une ouverture permettant au minimum le passage de l'air extérieur à travers la zone de stockage (ZS) ; par exemple les flacons pressurisés avec valve doseuse (Figure 23).

A partir de ce principe, différentes configurations de mise en oeuvre de l'appareil pour l'administration d'un aérosol médicamenteux peuvent être envisagées, l'aérosol pouvant être à base de liquide ou de poudre selon les configurations.

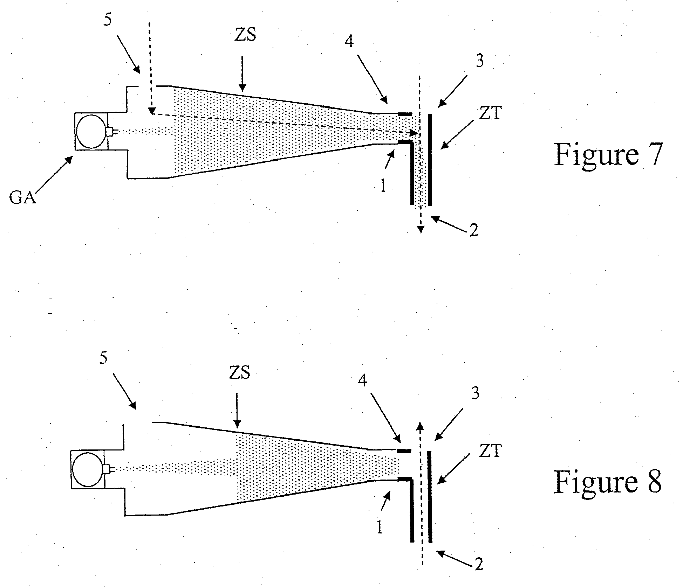

Une première configuration de l'appareil est illustrée aux figures 6, 7 et 8. Dans cette configuration, la zone de stockage (ZS) est utilisée horizontalement et la zone de transport (ZT) est connectée en son extrémité latérale. La zone de transport (ZT) possède trois ouvertures. Une première ouverture (1) est destinée à recevoir une zone de stockage (ZS) horizontale et forme un angle de 90° avec l'axe reliant les deux autres ouvertures de la zone de transport (ZT). La deuxième ouverture (2) de la zone de transport (ZT) est destinée à recevoir la bouche ou la face du patient. La troisième ouverture (3) placée dans l'axe de la deuxième ouverture (2) n'est connectée à aucun élément. L'ouverture (3) a la même surface que l'ouverture (1). La zone de stockage (ZS) possède au minimum deux ouvertures, la première (4) est destinée à être connectée avec l'ouverture (1) de la zone de transport (ZT), la deuxième (5) n'est connectée à aucun élément et est destinée à faire pénétrer l'air extérieur dans la zone de stockage (ZS). L'aérosol produit par un générateur d'aérosol (GA) est reçu dans la zone de stockage (ZS). Dans cette configuration, durant la phase inspiratoire (figure 7), la dépression créée par le patient fait pénétrer l'air extérieur par les ouvertures (3) et (5). L'air pénétrant dans la zone de stockage (ZS) par l'intermédiaire de l'ouverture (5) traverse la zone de stockage (ZS) pour transporter l'aérosol vers l'ouverture (4) de la zone de stockage (ZS) c'est-à-dire vers l'ouverture (1) de la zone de transport (ZT). L'aérosol est ensuite transporté depuis l'ouverture (1) vers le patient via l'ouverture (2).
Durant la phase expiratoire (figure 8), l'air expiré depuis l'ouverture (2) est expulsé directement vers l'ouverture (3) en prolongement. L'aérosol ne sort pas de la zone de stockage (ZS) à travers les ouvertures (4) ou (5) mais reste dans la zone de stockage (ZS) pour la prochaine inspiration.

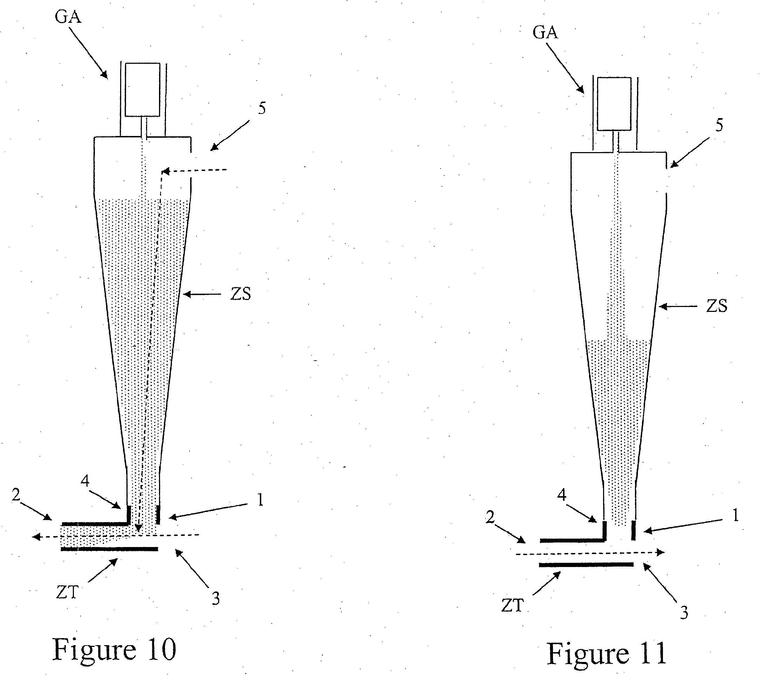

Une deuxième configuration de l'appareil est illustrée aux figures 9, 10 et 11. Dans cette configuration, la zone de stockage (ZS) est utilisée verticalement et la zone de transport (ZT) est connectée en son extrémité basse. La zone de transport (ZT) possède trois ouvertures. Une première ouverture (1) est destinée à recevoir une zone de stockage (ZS) verticale et forme un angle de 90° avec l'axe reliant les deux autres ouvertures (2-3) de la zone de transport (ZT). La deuxième ouverture (2) de la zone de transport (ZT) est destinée à recevoir la bouche ou la face du patient. La troisième ouverture (3) placée dans l'axe de la deuxième ouverture (2) n'est connectée à aucun élément. L'ouverture (3) a la même surface que l'ouverture (1). La zone de stockage (ZS) possède au minimum deux ouvertures, la première (4) est destinée à être connectée avec l'ouverture (1) de la zone de transport (ZT), la deuxième (5) n'est connectée à aucun élément et est destinée à faire pénétrer l'air extérieur dans la zone de stockage (ZS). L'aérosol produit par un générateur d'aérosol (GA) est reçu dans la zone de stockage (ZS). Dans cette configuration, durant la phase inspiratoire (figure 10), la dépression créée par le patient fait pénétrer l'air extérieur par les ouvertures (3) et (5). L'air pénétrant dans la zone de stockage (ZS) par l'intermédiaire de l'ouverture (5) traverse la zone de stockage (ZS) de haut en bas pour transporter l'aérosol vers l'ouverture (4) de la zone de stockage (ZS) c'est-à-dire vers l'ouverture (1) de la zone de transport (ZT). L'aérosol est ensuite transporté depuis l'ouverture (1) via l'ouverture (2) vers le patient.
Durant la phase expiratoire (figure 11), l'air expiré depuis l'ouverture (2) est expulsé directement vers l'ouverture (3). L'aérosol ne sort pas de la zone de stockage (ZS) à travers les ouvertures (4) ou (5) mais reste dans la zone de stockage pour la prochaine inspiration.

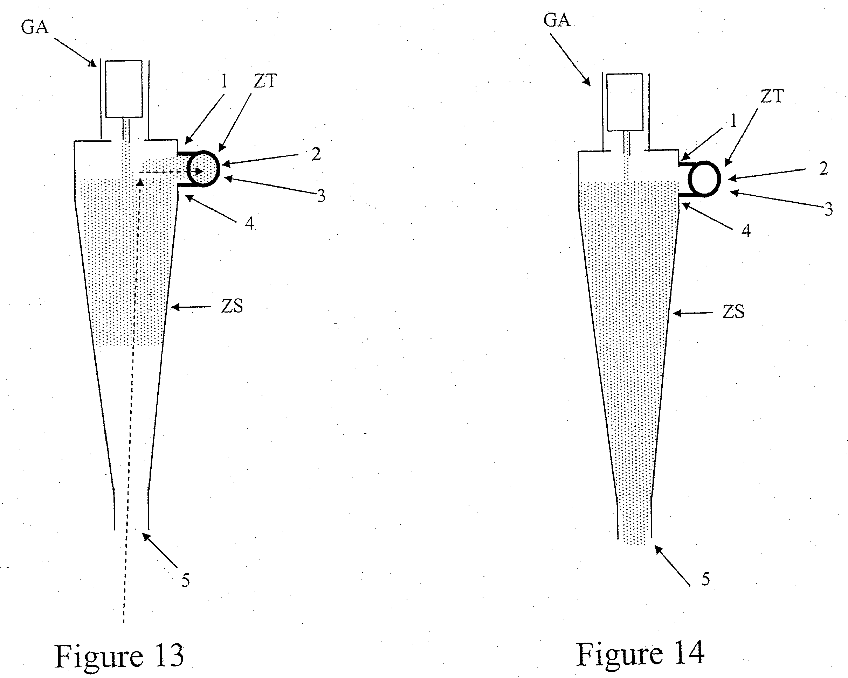

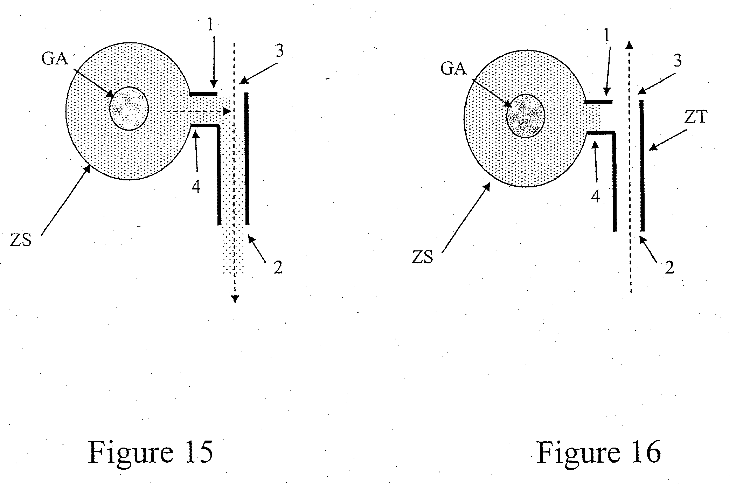

Une troisième configuration de l'appareil est illustrée aux figures 12, 13, 14, 15 et 16. Dans cette configuration, la zone de stockage (ZS) est utilisée verticalement et la zone de transport (ZT) est connectée sur le haut de sa face latérale. La zone de transport (ZT) possède trois ouvertures. Une première ouverture (1) est destinée à recevoir une zone de stockage (ZS) verticale et forme un angle de 90° avec l'axe reliant les deux autres ouvertures (2-3) de la zone de transport (ZT). La deuxième ouverture (2) de la zone de transport (ZT) est destinée à recevoir la bouche ou la face du patient. La troisième ouverture (3) placée dans l'axe de la deuxième ouverture (2) n'est connectée à aucun élément. L'ouverture (3) a la même surface que l'ouverture (1). La zone de stockage (ZS) possède au minimum deux ouvertures, la première (4) est destinée à être connectée avec l'ouverture (1) de la zone de transport (ZT), la deuxième (5) n'est connectée à aucun élément et est destinée à faire pénétrer l'air extérieur dans la zone de stockage (ZS). L'aérosol produit par un générateur d'aérosol (GA) est reçu dans la zone de stockage (ZS). Dans cette configuration, durant la phase inspiratoire (figures 13 et 15), la dépression créée par le patient fait pénétrer l'air extérieur par les ouvertures (3) et (5). L'air pénétrant dans la zone de stockage (ZS) par l'intermédiaire de l'ouverture (5) traverse la zone de stockage (ZS) pour transporter l'aérosol vers l'ouverture (4) de la zone de stockage (ZS) c'est-à-dire vers l'ouverture (1) de la zone de transport (ZT). L'aérosol est ensuite transporté depuis l'ouverture (1) vers le patient via l'ouverture (2).
Durant la phase expiratoire (figures 14 et 16), l'air expiré depuis l'ouverture (2) est expulsé directement vers l'ouverture (3). L'aérosol ne sort pas de la zone de stockage (ZS) à travers les ouvertures (4) ou (5) mais reste dans la zone de stockage pour la prochaine inspiration.

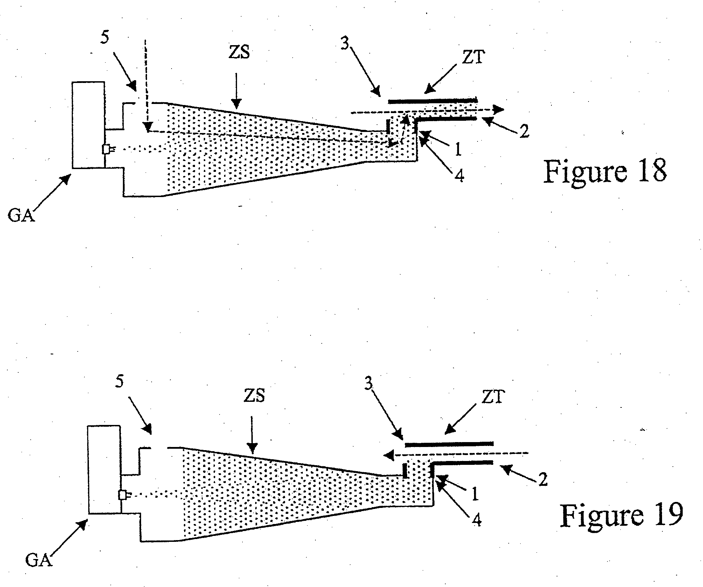

Une quatrième configuration de l'appareil est illustrée aux figures 17, 18 et 19. Dans cette configuration, la zone de stockage (ZS) est utilisée horizontalement et la zone de transport (ZT) est connectée sur sa face supérieure. La zone de transport (ZT) possède trois ouvertures. Une première ouverture (1) est destinée à recevoir une zone de stockage (ZS) verticale et forme un angle de 90° avec l'axe reliant les deux autres ouvertures (2-3) de la zone de transport (ZT). La deuxième ouverture (2) de la zone de transport (ZT) est destinée à recevoir la bouche ou la face du patient. La troisième ouverture (3) placée dans l'axe de la deuxième ouverture (2) n'est connectée à aucun élément. L'ouverture (3) a la même surface que l'ouverture (1). La zone de stockage (ZS) possède au minimum deux ouvertures, la première (4) est destinée à être connectée avec l'ouverture (1) de la zone de transport (ZT), la deuxième (5) n'est connectée à aucun élément et est destinée à faire pénétrer l'air extérieur dans la zone de stockage (ZS). L'aérosol produit par un générateur d'aérosol (GA) est reçu dans la zone de stockage (ZS). Dans cette configuration, durant la phase inspiratoire (figure 18) la dépression créée par le patient fait pénétrer l'air extérieur par les ouvertures (3) et (5). L'air pénétrant dans la zone de stockage (ZS) par l'intermédiaire de l'ouverture (5) traverse la zone de stockage (ZS) pour transporter l'aérosol vers l'ouverture (4) de la zone de stockage (ZS) c'est-à-dire vers l'ouverture (1) de la zone de transport (ZT). L'aérosol est ensuite transporté depuis l'ouverture (1) vers le patient via l'ouverture (2).
Durant la phase expiratoire (figure 19), l'air expiré depuis l'ouverture (2) est expulsé directement vers l'ouverture (3). L'aérosol ne sort pas de la zone de stockage (ZS) à travers la ou les ouvertures (4) ou (5) mais reste dans la zone de stockage pour la prochaine inspiration.

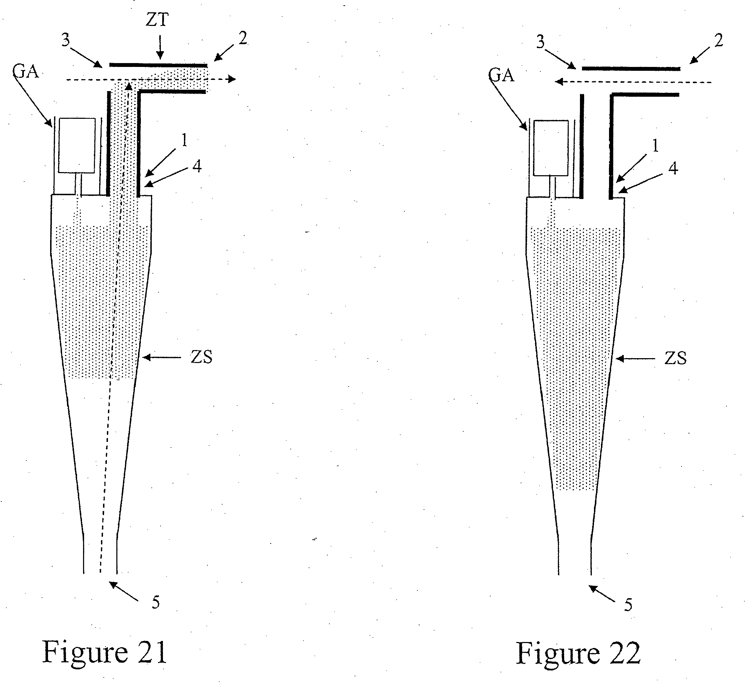

Une cinquième configuration de l'appareil est illustrée aux figures 20, 21 et 22. Dans cette configuration, la zone de stockage (ZS) est utilisée verticalement et la zone de transport (ZT) est connectée sur sa face supérieure. La zone de transport (ZT) possède trois ouvertures. Une première ouverture (1) est destinée à recevoir une zone de stockage (ZS) verticale et forme un angle de 90° avec l'axe reliant les deux autres ouvertures de la zone de transport (ZT). La deuxième ouverture (2) de la zone de transport (ZT) est destinée à recevoir la bouche ou la face du patient. La troisième ouverture (3) placée dans l'axe de la deuxième ouverture (2) n'est connectée à aucun élément. L'ouverture (3) a la même surface que l'ouverture (1). La zone de stockage (ZS) possède au minimum deux ouvertures, la première (4) est destinée à être connectée avec l'ouverture (1) de la zone de transport (ZT), la deuxième (5) n'est connectée à aucun élément et est destinée à faire pénétrer l'air extérieur dans la zone de stockage (ZS). L'aérosol produit par un générateur d'aérosol (GA) est reçu dans la zone de stockage (ZS). Dans cette configuration, durant la phase inspiratoire (figure 21) la dépression créée par le patient fait pénétrer l'air extérieur par les ouvertures (3) et (5). L'air pénétrant dans la zone de stockage (ZS) par l'intermédiaire de l'ouverture (5) traverse la zone de stockage (ZS) de bas en haut pour transporter l'aérosol vers l'ouverture (4) de la zone (ZS) c'est-à-dire vers l'ouverture (1) de la zone de transport (ZT). L'aérosol est ensuite transporté depuis l'ouverture (1) vers le patient via l'ouverture (2). Durant la phase expiratoire (figure 22), l'air expiré depuis l'ouverture (2) est expulsé directement vers l'ouverture (3). L'aérosol ne sort pas de la zone de stockage (ZS) à travers la ou les ouvertures (4) ou (5) mais reste dans la zone de stockage (ZS) pour la prochaine inspiration.

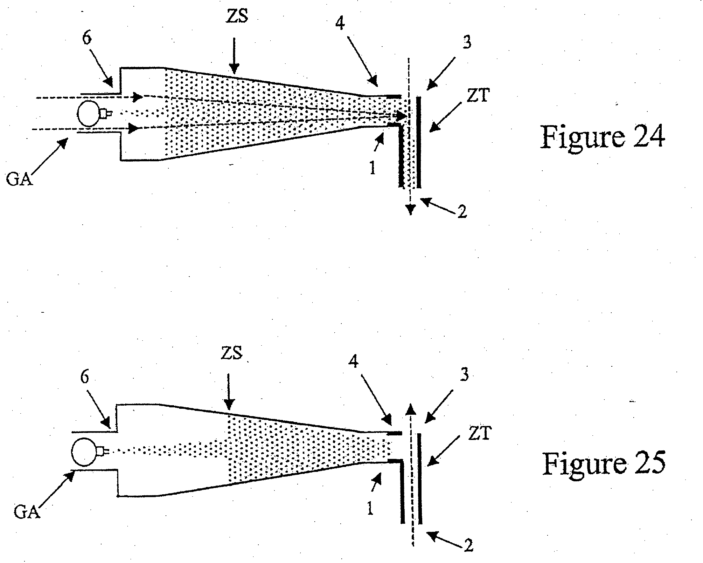

Une sixième configuration de l'appareil est illustrée aux figures 23, 24 et 25. Dans cette configuration, la zone de stockage (ZS) est utilisée horizontalement et possède uniquement deux ouvertures. La zone de transport (ZT) est connectée à l'extrémité latérale de la zone de stockage (ZS). La zone de transport (ZT) possède trois ouvertures. Une première ouverture (1) est destinée à recevoir une zone de stockage (ZS) horizontale et forme un angle de 90° avec l'axe reliant les deux autres ouvertures de la zone de transport (ZT). La deuxième ouverture (2) de la zone de transport (ZT) est destinée à recevoir la bouche ou la face du patient. La troisième ouverture (3) placée dans l'axe de la deuxième ouverture (2) n'est connectée à aucun élément. L'ouverture (3) a la même surface que l'ouverture (1). La zone de stockage (ZS) possède deux ouvertures, la première (4) est destinée à être connectée avec l'ouverture (1) de la zone de transport (ZT), la deuxième (6) est destinée à recevoir le générateur d'aérosol (GA) et à faire pénétrer l'air extérieur dans la zone de stockage (ZS) par l'intermédiaire du GA. L'aérosol produit par un générateur d'aérosol (GA) est reçu dans la zone de stockage (ZS). Dans cette configuration, durant la phase inspiratoire (figure 24), la dépression créée par le patient fait pénétrer l'air extérieur par les ouvertures (3) et (6). L'air pénétrant dans la zone de stockage (ZS) par l'intermédiaire de l'ouverture (6) du GA traverse la zone de stockage (ZS) pour transporter l'aérosol vers l'ouverture (4) de la zone de stockage (ZS) c'est-à-dire vers l'ouverture (1) de la zone de transport (ZT). L'aérosol est ensuite transporté depuis l'ouverture (1) vers le patient via l'ouverture (2). Durant la phase expiratoire (figure 25), l'air expiré depuis l'ouverture (2) est expulsé directement vers l'ouverture (3) en prolongement. L'aérosol ne sort pas de la zone de stockage (ZS) à travers les ouvertures (4) ou (6) mais reste dans la zone de stockage (ZS) pour la prochaine inspiration.

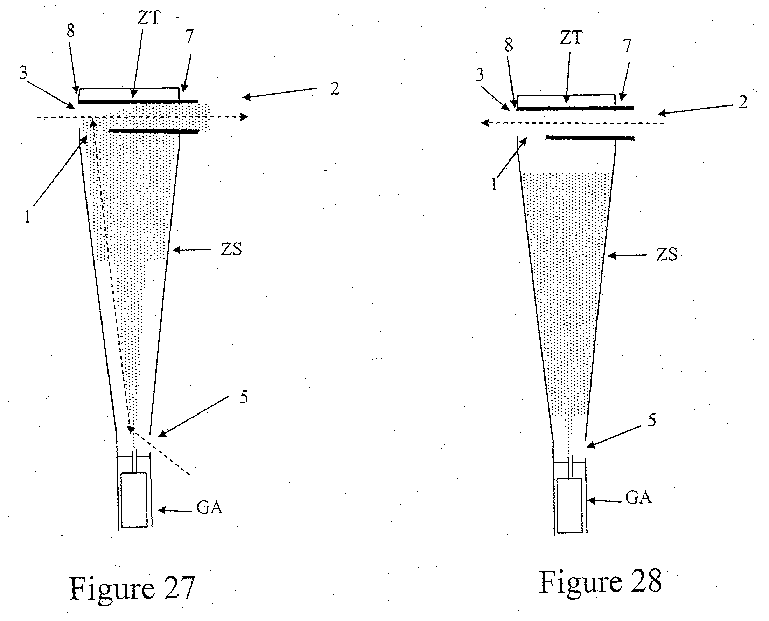

Une septième configuration de l'appareil, qui ne fait pas partie de la présente invention, est illustrée aux figures 26, 27 et 28. Dans cette configuration, la zone de stockage (ZS) est utilisée verticalement et la zone de transport (ZT) est contenue dans la partie supérieure de la zone de stockage (ZS). La zone de transport (ZT) possède trois ouvertures. Une première ouverture (1) est contenue dans la zone de stockage (ZS) verticale et forme un angle de 90° avec l'axe reliant les deux autres ouvertures de la zone de transport (ZT). La deuxième ouverture (2) de la zone de transport (ZT) est destinée à recevoir la bouche ou la face du patient. La troisième ouverture (3) placée dans l'axe de la deuxième ouverture (2) n'est connectée à aucun élément. L'ouverture (3) a la même surface que l'ouverture (1). La zone de stockage (ZS) possède au minimum trois ouvertures, la première (5) n'est connectée à aucun élément et est destinée à faire pénétrer l'air extérieur dans la zone de stockage (ZS), la deuxième (7) est traversée par l'ouverture (2) de la zone de transport (ZT), la troisième (8) est traversée par l'ouverture (3) de la zone de transport (ZT). L'aérosol produit par un générateur d'aérosol (GA) est reçu dans la zone de stockage (ZS). Dans cette configuration, durant la phase inspiratoire (figure 27) la dépression créée par le patient fait pénétrer l'air extérieur par les ouvertures (3) et (5). L'air pénétrant dans la zone de stockage (ZS) par l'intermédiaire de l'ouverture (5) traverse la zone de stockage (ZS) de bas en haut pour transporter l'aérosol vers l'ouverture (1) de la zone de transport (ZT). L'aérosol est ensuite transporté depuis l'ouverture (1) vers le patient via l'ouverture (2).
Durant la phase expiratoire (figure 28), l'air expiré depuis l'ouverture (2) est expulsé directement vers l'ouverture (3). L'aérosol ne sort pas de la zone de stockage (ZS) à travers l'ouverture (5) mais reste dans la zone de stockage (ZS) pour la prochaine inspiration.

Dans les configurations précitées, l'emplacement de la zone de transport (ZT) sur la zone de stockage (ZS) peut varier en position, les figures ayant été décrites et citées à titre d'exemple. L'angulation entre la zone de transport (ZT) et la zone de stockage (ZS) peut être établie dans une amplitude entre 45° et 135°.

Suivant l'une quelconque des configurations décrites précédemment, la solution apparaît extrêmement avantageuse, car selon les tests effectués, il a été mesuré que la dose d'aérosol inhalée par le patient est équivalente à un système utilisé avec des valves.

L'appareil selon l'invention peut être utilisé non limitativement comme suit :
- en association avec un embout buccal ou nasal pour administrer l'aérosol au patient,
- en association avec un masque facial pour administrer l'aérosol patient,
- en association avec un nébuliseur à membrane vibrante pour la production de l'aérosol,
- en association avec un nébuliseur pneumatique pour la production de l'aérosol,
- en association avec un nébuliseur ultrasonique pour la production de l'aérosol,
- en association avec un flacon pressurisé avec valve doseuse pour la production de l'aérosol.

## Revendications

1. Appareil pour l'administration d'un aérosol médicamenteux comprenant :
- un générateur d'aérosol médicamenteux (GA),
- une chambre d'inhalation sans valve constituant une zone de stockage (ZS) de l'aérosol produit notamment pendant la phase d'expiration du patient par le générateur d'aérosol (GA), et
- un dispositif de transfert d'aérosol sans valve constituant une zone de transport (ZT) de l'aérosol depuis la chambre d'inhalation vers le patient,
- la chambre d'inhalation (ZS) comprenant au minimum deux ouvertures dont la première (4) est obligatoirement destinée à être connectée au dispositif de transfert d'aérosol (ZT) et la deuxième (5) permet au minimum le passage de l'air extérieur à travers la chambre d'inhalation (ZS) lors de la phase inspiratoire du patient pour assurer le transfert de l'aérosol au patient,
**caractérisé en ce que**:
- le dispositif de transfert d'aérosol (ZT), situé entre la chambre d'inhalation (ZS) et le patient lors de l'utilisation de l'appareil, comprend au minimum trois ouvertures, une première ouverture (1) connectée à la première ouverture (4) de la chambre d'inhalation (ZS), une seconde ouverture (2) destinée à être connectée au patient, et une troisième ouverture (3) étant libre et disposée à l'opposé de la seconde ouverture (2) dans l'axe de celle-ci, ladite première ouverture (1) se trouvant positionnée entre, et en communication avec, la seconde et la troisième ouverture (2,3), l'axe de la première ouverture (1) formant un angle de 45° à 135 ° avec l'axe reliant les deux autres ouvertures (2,3) de manière à définir un coude dont la fonction est de guider et d'orienter les flux d'air en cas d'expiration et d'inspiration du patient de manière différenciée, la disposition des trois ouvertures (1, 2, 3) ayant pour fonction d'autoriser le transfert de l'air expiré par le patient entre la seconde ouverture (2) et la troisième ouverture (3) située dans l'axe rectiligne du dispositif de transfert d'aérosol (ZT), l'air expiré depuis la seconde ouverture (2) étant expulsé directement vers la troisième ouverture (3), et en cas d'inspiration par le patient, d'autoriser un flux d'air entrant par la troisième ouverture (3) et par la première ouverture (4) de la chambre d'inhalation (ZS).

2. Appareil selon la revendication 1, ***caractérisé en ce que*** la surface de la première ouverture (1) du dispositif de transfert d'aérosol (ZT) est égale à la surface de la troisième ouverture (3), *et **en ce que*** l'angle formé entre l'axe de la première ouverture (1) du dispositif de transfert d'aérosol (ZT) et l'axe reliant les deux autres ouvertures (2, 3) est de 90°.

3. Appareil selon l'une quelconque des revendications 1 et 2, ***caractérisé en ce que*** le dispositif de transfert d'aérosol (ZT) est placé sur une face à un endroit quelconque de la chambre d'inhalation (ZS).

4. Appareil selon l'une quelconque des revendications 1 à 3, *en association* avec un embout buccal ou nasal pour administrer l'aérosol au patient.

5. Appareil selon l'une quelconque des revendications 1 à 3, *en association* avec un masque facial pour administrer l'aérosol au patient.

6. Appareil selon l'une quelconque des revendications 1 à 3, ***caractérisé en ce que*** le générateur d'aérosol (GA) consiste en un nébuliseur à membrane vibrante.

7. Appareil selon l'une quelconque des revendications 1 à 3, ***caractérisé en ce que*** le générateur d'aérosol (GA) consiste en un nébuliseur pneumatique.

8. Appareil selon l'une quelconque des revendications 1 à 3, ***caractérisé en ce que*** le générateur d'aérosol (GA) consiste en un nébuliseur ultrasonique.

9. Appareil selon l'une quelconque des revendications 1 à 3, ***caractérisé en ce que*** le générateur d'aérosol (GA) consiste en un flacon pressurisé avec valve doseuse.

## Patentansprüche

1. Vorrichtung zur Verabreichung eines arzneimittelhaltigen Aerosols, Folgendes umfassend:
- einen Erzeuger (GA) eines arzneimittelhaltiges Aerosol,
- eine ventillose Inhalationskammer, die einen Vorhaltebereich (ZS) für das vom Erzeuger (GA) des arzneimittelhaltigen Aerosols erzeugte Aerosol insbesondere während der Ausatmungsphase des Patienten bildet, und
- eine ventillose Aerosolübertragungsvorrichtung, die einen Transportbereich (ZT) für das Aerosol aus der Inhalationskammer zum Patienten bildet,
- eine Inhalationskammer (ZS), die mindestens zwei Öffnungen aufweist, wovon die erste (4) zwingend dazu bestimmt ist, an die Aerosolübertragungsvorrichtung (ZT) angeschlossen zu sein, und die zweite (5) zumindest den Durchtritt von Außenluft durch die Inhalatianskammer (ZS) bei der Einatmungsphase des Patienten ermöglicht, um die Übertragung des Aerosols an den Patienten sicherzustellen,
**dadurch gekennzeichnet, dass**:
- die Aerosolübertragungsvorrichtung (ZT), die sich zwischen der Inhalationskammer (ZS) und dem Patienten befindet, bei der Benutzung der Vorrichtung mindestens drei Öffnungen aufweist, und zwar eine erste Öffnung (1), die an die erste Öffnung (4) der Inhalationskammer (ZS) angeschlossen ist, eine zweite Öffnung (2), die zum Anschluss an den Patienten bestimmt ist, und eine dritte Öffnung (3), die frei und der zweiten Öffnung (2) entgegengesetzt in deren Achse angeordnet ist, wobei die erste Öffnung (1) zwischen und in Verbindung mit der zweiten und dritten Öffnung (2, 3) stehend positioniert ist, wobei die Achse der ersten Öffnung (1) mit der die beiden anderen Achsen (2, 3) verbindenden Achse einen Winkel von 45° bis 135° bildet, so dass eine Krümmung gebildet ist, deren Aufgabe darin besteht, die Luftströme im Aus- und Einatmungsfall des Patienten differenziert zu leiten und auszurichten, wobei die Funktion der Anordnung der drei Öffnungen (1, 2, 3) darin besteht, die Übertragung der vom Patienten ausgeatmeten Luft zwischen der zweiten Öffnung (2) und der dritten Öffnung (3) zuzulassen, die sich in der geradlinigen Achse der Aerosolübertragungsvarrichtung (ZT) befindet, wobei die aus der zweiten Öffnung (2) ausgeatmete Luft direkt zur dritten Öffnung (3) hin ausgestoßen wird, und im Fall eines Einatmens des Patienten, einen Luftstrom zuzulassen, der durch die dritte Öffnung (3) und durch die erste Öffnung (4) der inhalationskamrner (ZS) eintritt.

2. Vorrichtung nach Anspruch 1, dadurch gekenntzeichnet, dass die Oberfläche der ersten Öffnung (1) der Aerosolübertragungsvorrichtung (ZT) gleich der Oberfläche dritten Öffnung (3) ist, und dass der Winkel, der zwischen der Achse der ersten Öffnung (1) der Aerosolübertragungsvorrichtung (ZT) und der die beiden anderen Öffnungen (2, 3) verbindenden Achse gebildet ist, 90° beträgt.

3. Vorrichtung nach einem der Ansprüche 1 und 2, **dadurch gekennzeichnet, dass** die Aerosolübertragungsvorrichtung (ZT) auf einer Fläche an einer beliebigen Stelle der Inhalationskammer (ZS) angeordnet ist.

4. Vorrichtung nach einem der Ansprüche 1 bis 3, in Verbindung mit einem Mund- oder Nasenstück zum Verabreichen des Aerosols an den Patienten.

5. Vorrichtung nach einem der Ansprüche 1 bis 3, in Verbindung mit einer Gesichtsmaske zum Verabreichen des Aerosols an den Patienten.

6. Vorrichtung nach einem der Ansprüche 1 bis 3, **dadurch gekennzeichnet**, das der Aerosolerzeuger (GA) aus einem Zerstäuber mit Vibrationsmembran besteht.

7. Vorrichtung nach einem der Ansprüche 1 bis 3, **dadurch gekennzeichnet, dass** der Aerosolerzeuger (GA) aus einem pneumatischen Zerstäuber besteht.

8. Vorrichtung nach einem der Ansprüche 1 bis 3, **dadurch gekennzeichnet, dass** der Aerosolerzeuger (GA) aus einem Ultraschallzerstäuber besteht.

9. Vorrichtung nach einem der Ansprüche 1 bis 3, **dadurch gekennzeichnet, dass** der Aerosolerzeuger (GA) aus einer Druckflasche mit Dosierventil besteht.

## Claims

1. An apparatus for the delivery of a medicinal aerosol comprising:
- a generator of medicinal aerosol (GA),
- a valve-less inhalation chamber forming an area (ZS) for storing the aerosol generated, in particular during the patient's exhalation phase, by the aerosol generator (GA), and
- a valve-less aerosol transfer device forming an area (ZT) for transporting the aerosol from the inhalation chamber to the patient,
- the inhalation chamber (ZS) comprising at least two openings, the first one (4) being necessarily intended to be connected to the aerosol transfer device (ZT) and the second one (5) allowing at least the flowing of outer air through the inhalation chamber (ZS) during the patient's inhalation phase to transfer the aerosol to the patient,
**characterized in that**
- the aerosol transfer device (ZT), located between the inhalation chamber (ZS) and the patient when the apparatus is being used, comprises at least three openings, a first opening (1) connected to the first opening (4) of the inhalation chamber (ZS), a second opening (2) intended to be connected to the patient, and a third opening (3) being free and arranged opposite to the second opening (2) in the axis thereof, said first opening (1) being positioned between and in communication with the second and the third openings (2, 3), the axis of the first opening (1) forming an angle in the range between 45° and 135° with the axis connecting the two other openings (2, 3) to define an elbow having the function of guiding and directing air flows in the case of the patient's exhalation and inhalation in a differentiated manner, the arrangement of the three openings (1, 2, 3) having the function of allowing the transfer of the air expired by the patient between the second opening (2) and the third opening (3) located in the rectilinear axis of the aerosol transfer device (ZT), the air expired from the second opening (2) being directly expelled towards the third opening (3), and in the case of an inspiration by the patient, to allow an air flow coming in through the third opening (3) and through the fourth opening (4) of the inhalation chamber (ZS).

2. The apparatus of claim 1, ***characterized in that*** the surface of the first opening (1) of the aerosol transfer device (ZT) is equal to the surface of the third opening (3), *and **in that*** the angle formed between the axis of the first opening (1) of the aerosol transfer device (ZT) and the axis connecting the two other openings (2, 3) is 90°.

3. The apparatus of any of claims 1 and 2, ***characterized in that*** the aerosol transfer device (ZT) is placed on a surface at any location of the inhalation chamber (ZS).

4. The apparatus of any of claims 1 to 3, *in association with* a mouth or nose piece to deliver the aerosol to the patient.

5. The apparatus of any of claims 1 to 3, *in association with* a face mask to deliver the aerosol to the patient.

6. The apparatus of any of claims 1 to 3, ***characterized in that*** the aerosol generator (GA) comprises a vibrating membrane nebulizer.

7. The apparatus of any of claims 1 to 3, ***characterized in that*** the aerosol generator (GA) comprises a pneumatic nebulizer.

8. The apparatus of any of claims 1 to 3, ***characterized in that*** the aerosol generator (GA) is an ultrasonic nebulizer.

9. The apparatus of any of claims 1 to 3, ***characterized in that*** the aerosol generator (GA) comprises a pressurized vial with a metering valve.
